# EUROPÄISCHE PATENTANMELDUNG

(11) **EP 0 969 319 A1**
(43) Veröffentlichungstag der Anmeldung: **05.01.2000**
(21) Anmeldenummer: 99112037.9
(22) Anmeldetag: 22.06.1999
(51) Int. Cl.: G03C 7/38, C07D 513/04

(54) **Farbfotographisches Silberhalogenidmaterial**

(30) Priorität: 04.07.1998 DE 19829978
(71) Anmelder: Agfa-Gevaert AG, 51373 Leverkusen (DE)
(72) Erfinder: Bergthaller, Peter Dr., 51467 Bergisch Gladbach (DE)

(57) **Zusammenfassung**

Gegenstand der vorliegenden Erfindung ist eine neue Verbindung der Formel (I) 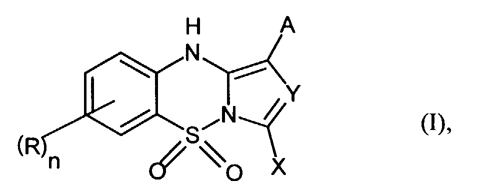 mit der Maßgabe, daß Y für N steht sowie ein farbfotografisches Silberhalogenidmaterial mit mindestens einer rotempfindlichen Silberhalogenidschicht, der erfindungsgemäß eine Verbindung der Formel (I) zugeordnet ist, sowie ein fotografisches Farbentwicklungsverfahren bei dem die Kuppler der Formel (I) mit Farbentwicklern vom Typ der Phenylendiamine und/oder Toluylendiamine zu Kupplungsprodukten umgesetzt werden, die insbesondere im Infrarotbereich eine starke Absorption zeigen. Ein weiterer Gegenstand betrifft die Verwendung von Verbindungen der Formel (I) als Blaugrün- und/oder Infrarotkuppler in farbfotografischen Silberhalogenidmaterialien.

## Beschreibung

Die Erfindung betrifft neue Verbindungen der Formel (I), mit der Maßgabe, daß Y für N steht sowie ein farbfotografisches Silberhalogenidmaterial mit mindestens einer rotempfindlichen Silberhalogenidschicht, der erfindungsgemäß eine Verbindung der Formel (I) zugeordnet ist, sowie ein fotografisches Farbentwicklungsverfahren bei dem die Kuppler der Formel (I) mit Farbentwicklern vom Typ der Phenylendiamine und/oder Toluylendiamine zu Kupplungsprodukten umgesetzt werden, die insbesondere im Infrarotbereich eine starke Absorption zeigen. Ein weiterer Gegenstand betrifft die Verwendung von Verbindungen der Formel (I) als Blaugrün- und/oder Infrarotkuppler in farbfotografischen Silberhalogenidmaterialien.

An die Farbkuppler sowie an die daraus durch chromogene Entwicklung erhaltenen Farbstoffe werden in der Praxis vielfache Anforderungen gestellt. So soll die Kupplungsgeschwindigkeit der Farbkuppler mit dem Oxidationsprodukt des Farbentwicklers groß sein und die Farbstoffbildung mit hoher Ausbeute erfolgen. Farbkuppler und die daraus erhaltenen Farbstoffe müssen gegenüber Licht, erhöhter Temperatur und Feuchtigkeit hinreichend stabil sein. Dies gilt sowohl für unbelichtetes als auch für verarbeitetes Material. Beispielsweise darf sich der in den Bildweißen des verarbeiteten Materials noch vorhandene restliche Kuppler nicht verfärben. Außerdem sollen die Farbstoffe gegenüber gasförmigen reduzierenden oder oxidierenden Agentien hinreichend beständig sein. Sie müssen ferner, sofern dies nicht ausdrücklich anders vorgesehen ist, diffusionsfest verankert sein und sollen sich bei der chromogenen Entwicklung möglichst in gelöster oder zumindest nicht in grobdisperser Form abscheiden, insbesondere soll ihre Absorption nicht durch Bildung von Farbstoffaggregaten getrübt oder kurzwellig verschoben werden. Außerdem dürfen die mechanischen Eigenschaften der Schichten durch die Einlagerung der Farbkuppler nicht beeinträchtigt werden.

Schließlich sollten die aus den Farbkupplern bei der chromogenen Entwicklung entstehenden Farbstoffe eine Absorptionskurve aufweisen, deren Maximum möglichst genau der Farbe des jeweiligen subtraktiven Teilbildes entspricht, und möglichst geringe Nebenabsorptionen zeigen. In Colorfilmen sollen die Absorptionsmaxima der Bildfarbstoffe außerdem möglichst genau mit den Sensibilisierungsmaxima der zum Kopieren verwendeten Colorprintmaterialien übereinstimmen.

Als Blaugrünkuppler, d.h. als Farbkuppler, die zur Erzeugung des blaugrünen Teilbildes geeignet sind, werden im allgemeinen Verbindungen verwendet, die sich vom Phenol oder vom α-Naphthol ableiten. Daneben sind auch Blaugrünkuppler vom Imidazoltyp bekannt. Unter den Farbkupplern mit einem unkondensierten Imidazolring sind beispielsweise 2,4-disubstituierte sowie 4,5-disubstituierte Imidazole insbesondere aus folgenden Schriften bekannt: GB 1,545,507, EP 0 249 453, EP 0 304 856 und US 5,051,347. Die bekannten Blaugrünkuppler besitzen jedoch die zuvor genannten Merkmale nicht in ausreichendem Maße. Besonders das Absorptionsverhalten im Infrarotbereich der im Rahmen der konventionellen Entwicklung gebildeten Farbstoffe aus den Blaugrünkupplern des Standes der Technik ist nicht zufriedenstellend. Desweiteren ist auch die Kupplungsfähigkeit häufig noch nicht ausreichend. Es besteht daher weiterhin Bedarf an Blaugrünkupplern, die möglichst viele der zuvor aufgeführten Eigenschaften, sowie insbesondere solche, deren Kupplungsprodukte eine Absorption auch im Infrarotbereich aufweisen. Aus dem Stand der Technik sind Farbkuppler aus der Reihe der 1-Naphthol-2-carbonsäureamide bekannt, die nach Kupplung mit konventionellen Entwicklern Farbstoffe mit einer in den Infrarotbereich hineinreichenden Absoption bilden.

Daneben sind beispielsweise aus der DE 40 16 418 Farbkuppler aus der Klasse der Imidazo[5,1-c]benzothiadiazin-S,S-dioxide als fotografische Farbkuppler zur Erzeugung violetter bis blaugrüner Farbbilder bekannt. Die Kuppler sind jedoch schwerlöslich und die erhaltenen Azomethinfarbstoffe zeigen Nebenabsorptionen.

Der vorliegenden Erfindung liegt die Aufgabe zugrunde Blaugrünkuppler zur Verfügung zu stellen, welche sich nach Kupplung mit den üblichen Entwickleroxidationsprodukten durch eine besonders intensive Absorption im Infrarotbereich auszeichnen insbesondere durch eine Absorption im Bereich von 680 bis 750 nm. Gegenüber den aus dem Stand der Technik bekannten Blaugrünkupplern sollte nach Kupplung mit konventionellen Entwicklern eine langwellige Verschiebung der Absorptionsbande erreicht werden. Gleichzeitig sollten die Kuppler sich gut in das fotografische Silberhalogenidmaterial einarbeiten lassen und sich dort durch eine hohe Beständigkeit insbesondere gegenüber Licht auszeichnen.

Überraschenderweise wurde gefunden gefunden, daß sich die nachstehend beschriebenen Verbindungen der Formel (I) hervorragend als Blaugrün- bzw. Infrarotkuppler eignen und insbesondere die mit konventionellen Entwicklern gebildeten Farbstoffe im Infrarot eine deutliche Absorption aufweisen. Die als Ergebnis der chromogenen Kupplung mit Entwicklern vom Phenylendiamin- und/ oder Toluylendiamin-Typ erhaltenen Farbstoffe weisen Absorptionsmaxima im Bereich von 680 bis 750 nm, insbesondere von 700 bis 740 nm auf Gleichzeitig verfügen die Kuppler selber sowie die erhaltenen Blaugrün- und/oder Infrarotfarbstoffe über eine hohe Beständigkeit gegen Licht und Feuchtigkeit.

Gegenstand der vorliegenden Erfindung sind Verbindung der Formel (I) 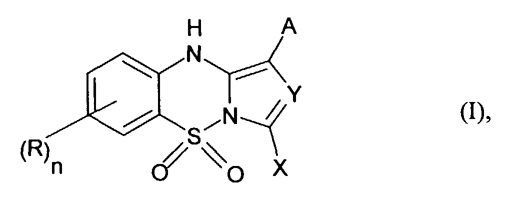 worin
- A: eine Elektronenakzeptorgruppe mit einem σₚ₋ Wert von >0,25,
- R: einen Substituenten,
- n: 0,1,2,3 oder 4,
- Y: N und
- X: H oder eine bei der chromogenen Kupplung abspaltbare Gruppe
bedeuten. Diese Verbindungen eignen sich beispielsweise zum Einsatz in fotografischen Materialen oder auch als Kuppler zur Herstellung von Azofarbstoffen oder Methinfarbstoffen, indem sie z.B. mit Diazoniumsalzen oder Aldehyden umgesetzt werden.

Ein weiterer Gegenstand der vorliegenden Anmeldung ist ein farbfotografisches Silberhalogenidmaterial mit wenigstens einer blauempfindlichen, wenigsten einen Gelbkuppler enthaltenden Silberhalogenidemulsionsschicht, wenigstens einer grünempfindlichen, wenigstens einen Purpurkuppler enthaltenden Silberhalogenidemulsionsschicht und wenigstens einer rotempfindlichen wenigstens einen Blaugrünkuppler enthaltenden Silberhalogenidemulsionsschicht, dadurch gekennzeichnet, daß der rotempfindlichen Schicht eine Verbindung der Formel (I) zugeordnet ist, 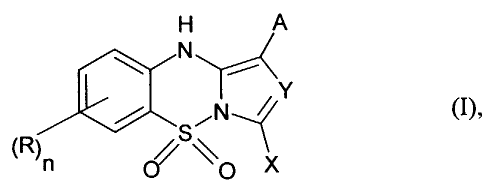 worin
- A: eine Elektronenakzeptorgruppe mit einem σₚ₋ Wert von >0,25,
- R: einen Substituenten,
- n: 0,1,2,3 oder 4,
- Y: N oder C-CN und
- X: H oder eine bei der chromogenen Kupplung abspaltbare Gruppe
bedeuten.

Besonders bevorzugt sind dabei Verbindungen, in denen Y für N steht.

Vorzugsweise bedeutet n 0,1 oder 2. Es kann sich dabei um die gleichen oder verschiedene Substituenten handeln. Unter einem Substituenten im Sinne der vorliegenden Anmeldung sind beispielsweise Halogene, wie insbesondere F, Cl oder Br zu verstehen, desweiteren Alkoxy-, Alkylthio-, Aryloxy-, Arylthio-, Acylamino-, Alkylsulfonamido-, Alkylsulfamoyl-, Alkylcarbamoyl-, Arylsulfonamido-, Arylsulfamoyl-, Arylcarbamoyl-, Alkyl-, Alkenyl-, Aryl-, Hetaryl-, Arylen-, Hetarylen-, Alkylen-, Alkoxycarbonyl-, Ureido- oder Cyangruppen. Bevorzugte Beispiele der durch **R** repräsentierten Gruppen sind: -CN, -Cl, -OR¹, -NR²CO-O R¹, -CONH R¹, - COO R¹, -S R¹. R¹ kann im Sinne der vorliegenden Erfindung für eine Alkyl-, Aryl-, Arylen-, Hetaryl- oder Hetarylengruppe stehen. Besonders bevorzugt handelt es sich bei R¹ um eine Methyl-, Ethyl-, Propyl-, Isopropyl-, Butyl-, Isobutyl- oder eine sekundär verzweigte Alkylgruppe mit 6 bis 30 C-Atomen, insbesondere **perflourierte Alkylgruppen** wie beispielsweise Triflourmethyl, Pentaflourethyl, Heptaflourpropyl und Nonaflourbutyl sind bevorzugt. Weiterhin bevorzugt sind substituierte sowie nicht substituierte Cycloalkyl-, insbesondere Cyclohexangruppen und auch substituierte sowie nicht substituierte Aryl-, insbesondere Phenylgruppen. R² steht vorzugsweise für H oder einen Alkylrest.

Unter **Alkyl** im Sinne der vorliegenden Anmeldung sind lineare oder verzweigte, cyclische oder geradkettige, substituierte oder nicht substituierte Kohlenwasserstoffgruppen zu verstehen vorzugsweise handelt es sich um Alkylgruppen mit 1 bis 20 C-Atomen, als offenkettige Alkylgruppen kommen insbesondere Methyl-, Ethyl-, n-Propyl-,n- Butyl-, n-Decyl-, n-Dodecyl-, n-Tetradecyl, n-Hexadecyl, n-Octadecyl sowie als verzweigte Alkylreste insbesondere 2-Hexyl-decyl und 2-Ethylhexylreste in Frage. Unter den Cycloalkylgruppen sind Cyclohexyl, insbesondere 4-t-butyl-Cyclohexyl, 2,6-di-t-butyl-4-methylcyclohexyl bevorzugt.

Unte**r Alkenyl** im Sinne der vorliegenden Erfindung sind lineare oder verzweigte, cyclische oder geradkettige, substituierte oder nicht substituierte ungesättigte Kohlenwasserstoffreste zu verstehen wie beispielsweise Ethenyl, 2-Propenyl, Isopropenyl.

Unter **Aryl** im Sinne der vorliegenden Anmeldung sind aromatische Kohlenwasserstoffgruppen zu verstehen, wobei es sich vorzugsweise um 5- oder 6-gliedrige Ringsysteme handelt, welche monocyclisch aber auch als kondensierte Ringsysteme vorliegen können. Es kann sich dabei sowohl um substituierte als auch um nicht substituierte Ringsysteme handeln. Besonders bevorzugt sind beispielsweise Phenyl- und Naphthylgruppen.

Unter **Hetaryl** sind im Sinne der vorliegenden Anmeldung aromatische Systeme zu verstehen, welche mindestens ein Heteroatom enthalten. Es handelt sich auch hierbei vorzugsweise um 5- oder 6-gliedrige Ringsysteme, welche monocyclisch aber auch als kondensierte Ringsysteme vorliegen können. Es kann sich dabei sowohl um substituierte als auch um nicht substituierte Ringsysteme handeln. Als Heteroatome kommen dabei insbesondere N, S und O in Frage. Ein Ringsystem kann vorzugsweise zwischen 1 und 3 Heteroatome aufweisen, wobei es sich um die gleichen oder verschiedene Heteroatome handeln kann. Bei den kondensierten Ringsystemen können mehrere gleiche oder verschiedene heterocyclische Systeme kondensiert sein, als auch Hetaryle mit Arylen. Typische Beispiele sind Pyridin, Pyridazin, Pyrimidin, Pyrazine, Oxazol, Isoxazol, Thiazole, 1,3,4-Oxdiazol, 1,2,4-Oxdiazol, Imidazol, 1,2,3-Triazol und 1,2,4-Triazol.

Erfindungsgemäß weist eine durch **A** dargestellte Elektronenakzeptorgruppe einen Wert der Hammet Substituenten Konstante σₚ ( zur Definition siehe Hansch et al. J. Med. Chem. 1973, 16, 1207 und ibid. 1977, 20, 304) von mehr als 0,25, vorzugsweise mehr als 0,3 und insbesondere mehr als 0,45 auf Derartige Substituenten sind beispielsweise eine Ketogruppe, eine Acetalgruppe, eine Carbonsäureestergruppe, eine Carbonamidgruppe, eine Cyangruppe, eine Sulfonamidgruppe, eine Acylharnstoffgruppe, eine Sulfonylcarbamoylgruppe, eine Sulfamoylcarbamoylgruppe, eine offene oder cyclische Amidingruppe, eine cyclische Amidrazongruppe, ein über ein C-Atom oder über ein N-Atom gebundener heterocyclischer Rest mit Elektronenakzeptorcharakter, z.B. eine Pyridylgruppe, eine Pyridazinylgruppe, eine Pyrimidinylgruppe, eine Triazinylgruppe, eine Pyrazinylgruppe, ein Imidazolrest, ein Triazolrest, ein Oxazolrest, ein 1,2,4-Oxdiazolrest oder ein 1,3,4-Oxdiazolrest, ein Thiophenrest, ein Thiazolrest, ein Thiazolonrest, ein Thiadiazolrest, ein Phosphonsäureesterrest, eine Sulfoxidgruppe, eine Sulfongruppe, eine Sulfilimingruppe, eine Sulfoximingruppe sein. Besonders bevorzugt handelt es sich bei der durch A dargestellten Gruppe um eine Alkoxycarbonyl-, Carbamoyl- oder eine Cyangruppe.

Erfindungsgemäß geeignete Substituenten **X** sind beispielsweise H oder organische Gruppen, die in der Regel über ein Schwefel-, Sauerstoff- oder Stickstoffatom an die Kupplungsstelle angeknüpft sind, wobei der abgespaltene Rest spezielle fotografische Wirkungen hervorrufen kann. Falls es sich bei der abspaltbaren Gruppe um eine cyclische Gruppe handelt, kann die Anknüpfung an die Kupplungsstelle des Kupplermoleküls entweder direkt über ein Atom, das Bestandteil eines Ringes ist, z.B. ein Stickstoffatom, oder indirekt über ein zwischengeschaltetes Glied erfolgen. Derartige abspaltbare Gruppen sind in großer Zahl bekannt, z.B. als Fluchtgruppen von Zweiäquivalentkupplern. Die abgespaltene Gruppe kann auch selbst ein Farbkuppler oder Weißkuppler sein.

Beispiele von über Sauerstoff angeknüpften abspaltbaren Gruppen entsprechen der Formel

-OR³,

worin R³ für einen acyclischen oder cyclischen organischen Rest steht, z.B. für Alkyl, Aryl, eine heterocyclische Gruppe oder Acyl, das sich beispielsweise von einer organischen Carbonsäure oder Sulfonsäure ableitet. Bei besonders bevorzugten abspaltbaren Gruppen dieser Art bedeutet R³ eine gegebenenfalls substituierte Phenylgruppe. Solche Gruppen sind beispielsweise in US-A 3,408,194 und DE-A 24 56 076 beschrieben.

Beispiele von über Stickstoff angeknüpften abspaltbaren Gruppen sind in den folgenden deutschen Offenlegungsschriften beschrieben:
DE-A 20 57 941, DE-A 21 63 812, DE-A 22 13 461, DE-A 22 19 917, DE-A 22 61 361, DE-A 22 63 875, DE-A 23 18 807, DE-A 23 29 587, DE-A 23 44 155, DE-A 23 63 675, DE-A 24 33 812, DE-A 24 41 779, DE-A 24 42 703, DE-A 25 28 638, DE-A 25 28 860, DE-A 26 37 817, DE-A 28 18 373, DE-30 20 416.

Hierbei handelt es sich durchwegs um 5- oder 6-gliedrige heterocyclische Ringe, die über ein Ringstickstoffatom mit der Kupplungsstelle des Kupplers verbunden sind. Die heterocyclischen Ringe enthalten vielfach benachbart zu dem die Bindung an das Kupplermolekül vermittelnden Stickstoffatom die Acidität erhöhende Gruppen, z.B. Carbonyl- oder Sulfogruppen oder Doppelbindungen. Ein typisches Beispiel dafür sind die Hydantoine.

Ein durch **X** dargestellter bei der chromogenen Entwicklung abspaltbarer Rest kann erfindungsgemäß vorzugsweise H, Halogen, insbesondere Cl, Br, F, oder ein Alkoxy-, Aryloxy-, Hetaryloxy-, Acyloxy-, Sulfonyloxy-, Alkoxycarbonyloxy-, Aryloxy-carbonyloxy-, Alkylthio-, Arylthio-, Hetarylthio-, Alkoxycarbonylthio-, Acylalkyl-amino-, Alkoxycarbonylamino-, N-Alkylsulfonamido-, Aryloxycarbonylamino- oder Carboxysubstituent sein, sowie ein über ein Stickstoffatom gebundener heterocyclischer Rest oder eine Arylazo- oder Hetarylazogruppe.

Ebenfalls bevorzugt kann eine in Formel (I) durch X dargestellte unter den Bedingungen der chromogenen Entwicklung abspaltbare Gruppe der Rest einer fotografisch wirksamen Verbindung sein. Im Sinne der vorliegenden Erfindung sind unter fotografisch wirksamen Verbindungen beispielsweise folgende zu verstehen: Entwicklungsinhibitoren, Bleichinhibitoren, Bleichbeschleuniger, Entwicklungsbeschleuniger, ein silberkeimbildendes "Nukleierungsmittel", eine lösliche ausentwicklungsfördernde Mercaptanverbindung, Stabilisatoren, Weißkuppler, Scavenger, eine zur Elektronenübertragung geeignete Hilfsentwicklersubstanz, z.B. vom Phenidontyp, oder Farb- oder Weißkupplerverbindungen.

Als Entwicklungsinhibitoren sind insbesondere solche aus der Reihe der Benzotriazole, der Thienotriazole, der monocyclischen 1,2,3-Triazole, der monocyclischen 1,2,4-Triazole, der 1-Aryl-5-mercaptotetrazole, der 1-Alkyl-5-mercaptotetrazole, der 2-Mercapto-5-alkylthio-1,3,4-thiadiazolsowie der 2-Mercapto-5-alkylthio-1,3,4-oxadiazole zu nennen.

Daneben kann X jedoch auch für eine Gruppe stehen, die sich aus einem sogenannten Zeitsteuerglied und dem daran gebundenen Rest einer fotografisch wirksamen Verbindung zusammensetzt. Als Zeitsteuerglied bezeichnet man eine Gruppe, die nach Abspaltung aus der Kupplungsstelle des Kupplers bei dessen Kupplung mit dem Oxidationsprodukt des Farbentwicklers befähigt ist, in einer Folgereaktion einen Rest einer fotografisch wirksamen Verbindung freizusetzen. Der daran gebundenene Rest der fotografisch wirksamen Verbindung wird in vielen Fällen verzögert freigesetzt und wirksam.

Bekannte Zeitsteuerglieder sind
- die in DE-A-2 803 145 beschriebenen Gruppen -O-CH(R)-, in denen das O-Atom an die Kupplungsstelle des Kupplers und das C-Atom an ein N-Atom einer fotografisch nützlichen Gruppe gebunden ist, welche nach Abspaltung vom Kuppler in einer raschen intramolekularen oder intermolekularen nukleophilen Verdrängungsreaktion freigesetzt werden kann (z.B. DE-A 2 855 697);
- Gruppen, in denen nach Abspaltung vom Kuppler eine Elektronenübertragung entlang eines konjugierten Systems stattfinden kann, wodurch ebenfalls die Freisetzung der fotografisch nützlichen Gruppe ausgenützt wird (z.B. in DE-A 3 105 026)
- oder eine Gruppe -V-C(=NR)-, worin V (z.B. -O-) an die Kupplungsstelle des Kupplers und das C-Atom an ein Atom der fotografisch nützlichen Verbindung gebunden ist und worin R beispielsweise für Aryl steht (z.B. in EP-A 0 127 063).

Das Zeitsteuerglied kann auch eine Gruppe sein, die nach Abspaltung aus der Kupplungsstelle des Kupplers selbst eine Redoxreaktion oder eine Kupplungsreaktion eingehen kann und als Folge einer solchen Reaktion ihrerseits eine an sie gebundene fotografisch nützliche Gruppe freisetzt.

Die erfindungsgemäß einzusetzenden Farbkuppler sind bevorzugt mit einem Ballastrest ausgestattet. Als Ballastreste sind solche Reste anzusehen, die es ermöglichen, die erfindungsgemäßen Farbkuppler in den üblicherweise verwendeten hydrophilen Bindemitteln diffusionsfest einzulagern. Hierzu sind vorzugsweise organische Reste geeignet, die im allgemeinen geradkettige oder verzweigte aliphatische Gruppen und gegebenenfalls auch carbocyclische oder heterocyclische aromatische Gruppen die im allgemeinen 8 bis 20, vorzugsweise 12 bis 18 C-Atomen enthalten. In der Formel (I) kann beispielsweise R einen Ballastrest darstellen, bevorzugt ist der Ballastrest jedoch Bestandteil der Gruppe A. Insbesondere bevorzugte Ballastreste sind dabei offenkettige oder cyclische, verzweigte oder unverzweigte Kohlenwasserstoffe mit 12 bis 18 C-Atomen.

Beispiele für erfindungsgemäß zu verwendende Blaugrün- und/oder Infrarotkuppler sind im folgenden aufgeführt.

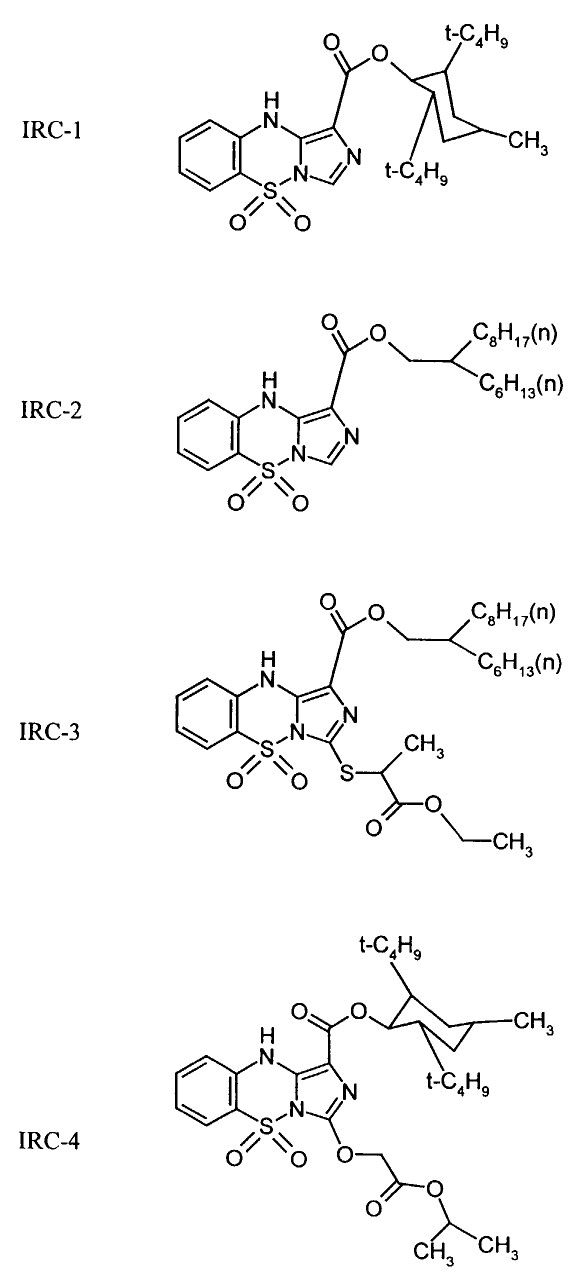

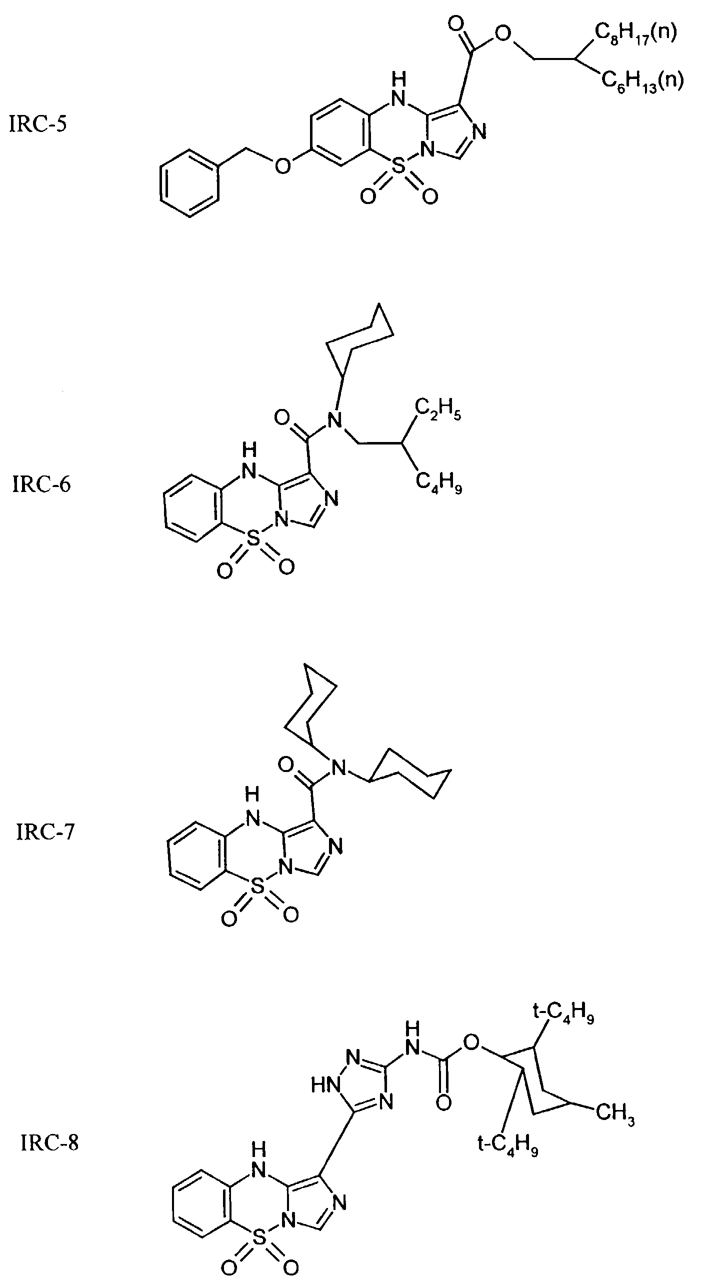

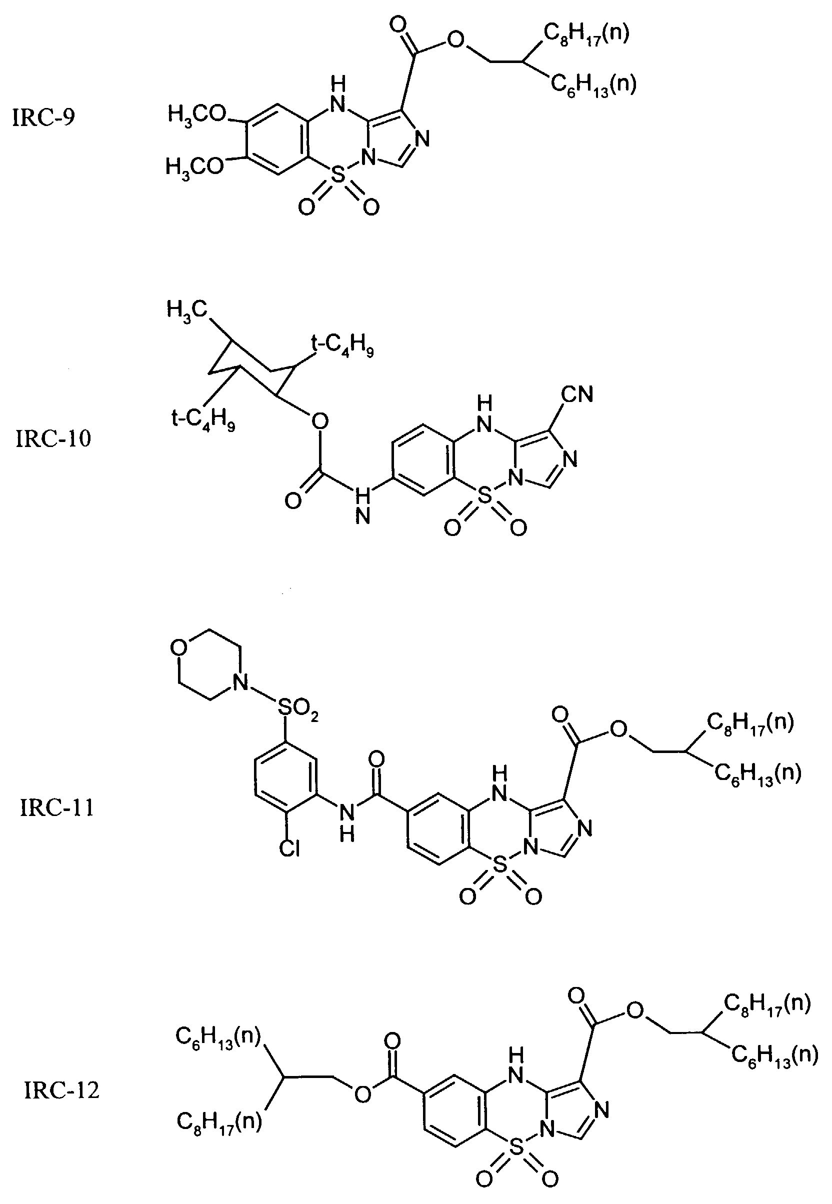

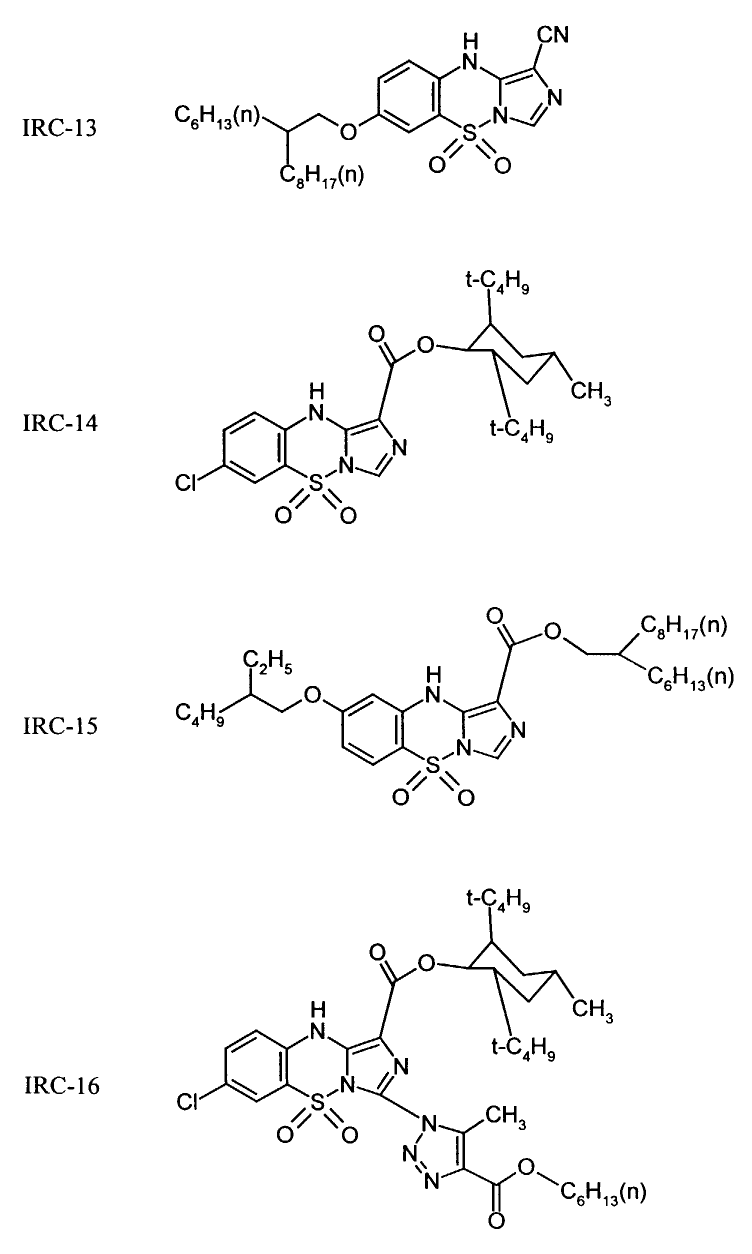

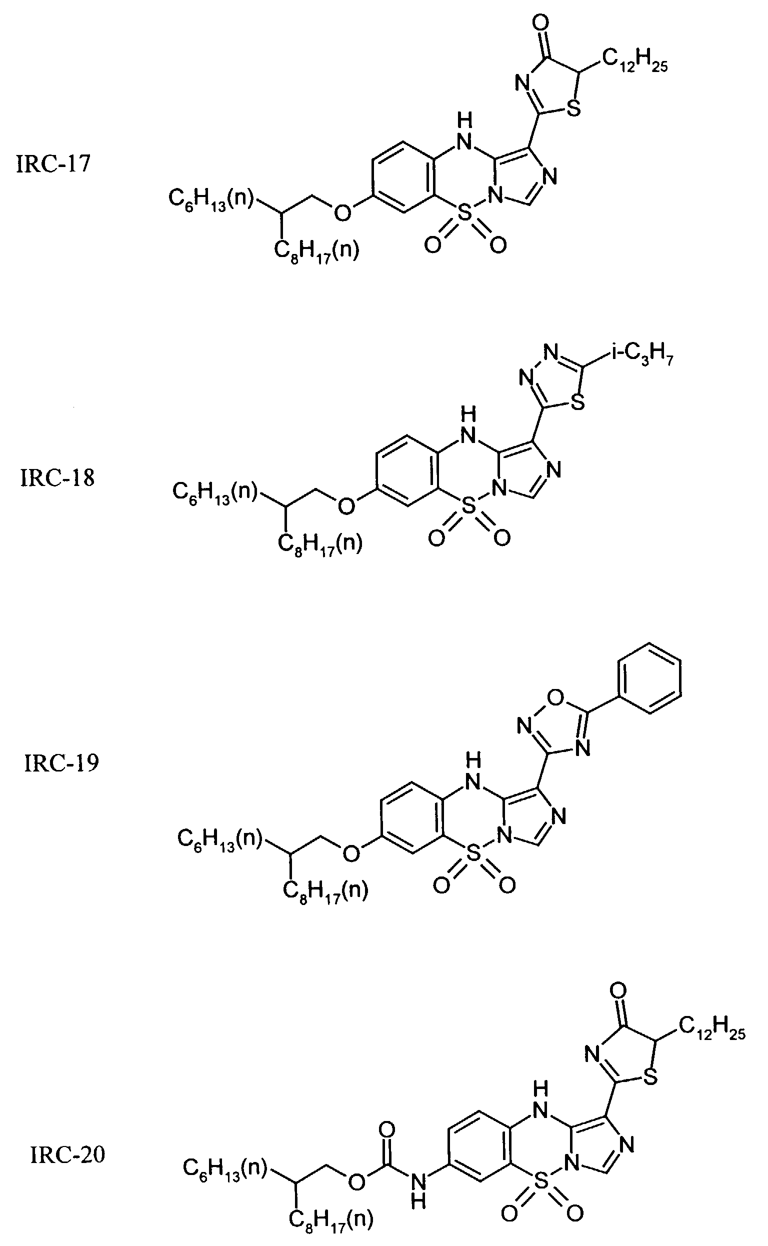

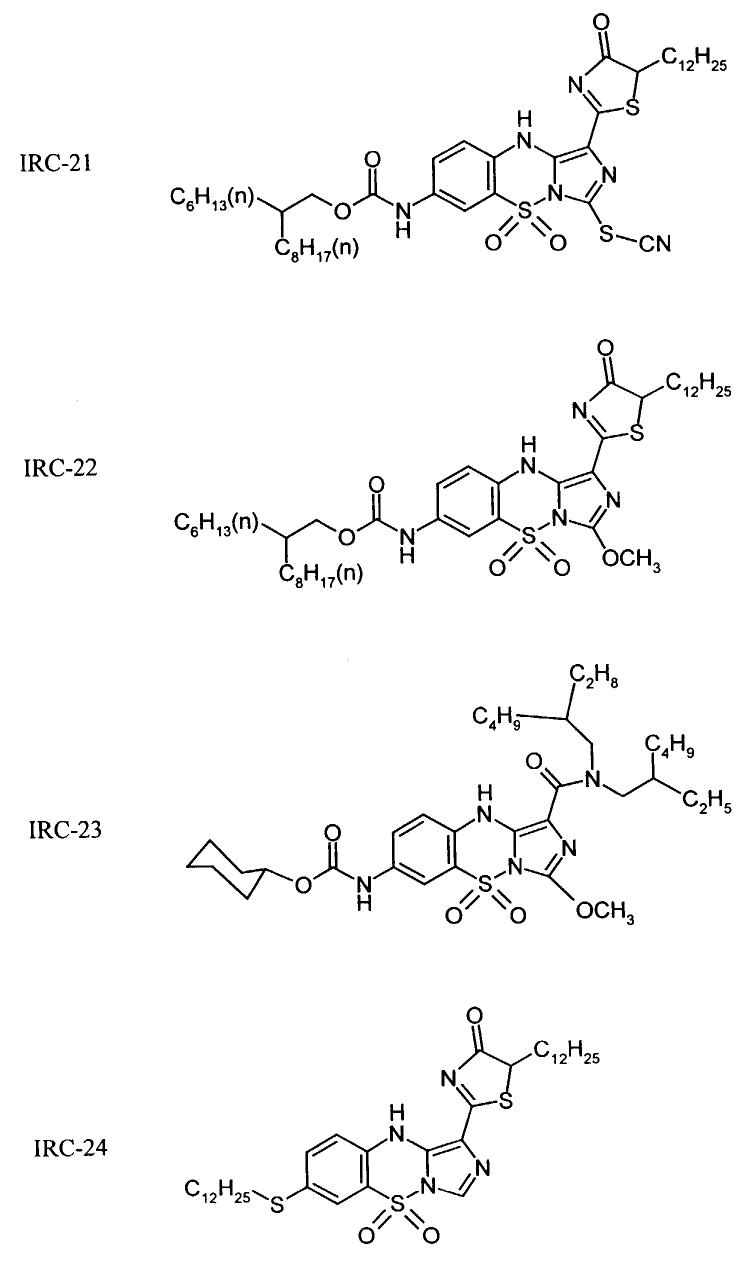

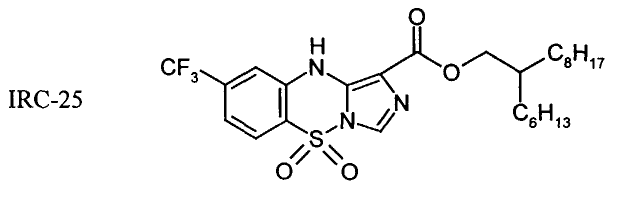

Gegenüber den aus der DE 40 16 418 bekannten und gemäß Formel (25) und (26) offenbarten Blaugrünkupplern (Imidazo[5,1-c]benzothiadiazin-S,S-dioxide) zeichnen sich die erfindungsgemäßen Imidazo[5,1-b]benzothiadiazin-S,S-dioxide durch eine höhere Stabilität und eine bessere Kupplungsfähigkeit, sowohl durch eine höhere Kupplungsgeschwindigkeit, als auch durch einen besseren Umsatz, aus. Die aus den neuen Farbkupplern zugänglichen Azomethinfarbstoffe zeigen insbesondere eine längerwellig absorbierende Hauptbande im Bereich von 680 bis 750 nm.

Zur Synthese der erfindungsgemäßen Farbkuppler stehen je nach der Konstitution und nach den Substituenten und insbesondere nach dem angegliederten Ring mehrere Methoden zur Verfügung. Insbesondere hat sich herausgestellt, daß die in DB 40 16 418 beschriebenen Cyclisierungsverfahren geeignet sind, sofern man nicht, wie dort beschrieben alkalisch aufarbeitet, sondern im Sauren. Werden die bei dem in der DE 40 16 418 beschriebenen Verfahren freigesetzten großen Mengen an Chlorwasserstoff nicht durch säurebindende Mittel gebunden, so erhält man die Verbindungen gemäß der Formel (I). Gegenüber dem in der DE 40 16 418 beschriebenen Verfahren ist es zur Herstellung der erfindungsgemäßen Kuppler entscheidend, daß die Aufarbeitung (währenddessen der Ringschluß erfolgt) stets unter sauren Bedingungen erfolgt. Vorzugsweise sollte ein pH-Wert < 3 insbesondere <2 eingehalten werden.

Das erfindungsgemäße farbfotografische Material kann weiterhin Verbindungen enthalten, die beispielsweise einen Entwicklungsinhibitor, einen Entwicklungsbeschleuniger, einen Bleichbeschleuniger, einen Entwickler, ein Silberhalogenidlösungsmittel, ein Schleiermittel oder ein Antischleiermittel in Freiheit setzten können, beispielsweise sogenannte DIR-Hydrochinone oder andere Verbindungen, wie sie beispielsweise in US-A 4,636,546, US-A 4,345,024, US-A 4,684,604 und in DE-A 24 47 079, DE-A 25 15 213 und DE-A 31 45 640 oder in EP-A 198 438 beschrieben sind. Diese Verbindungen erfüllen die gleiche Funktion wie DIR-, DAR- oder FAR-Kuppler, abgesehen davon, daß sie keine Kupplungsprodukte bilden.

Hochmolekulare Farbkuppler sind beispielsweise in DE-C 1 297 417, DE-A 24 07 569, DE-A 31 48 125, DE-A 32 17 200, DE-A 33 20 079, DE-A 33 24 932, DE-A 33 31 743, DE-A 33 40 376, EP-A 27 284 und US-A 4 080 211 beschrieben. Die hochmolekularen Farbkuppler werden in der Regel durch Polymerisation von ethylenisch ungesättigten Farbkupplermonomeren hergestellt. Sie können aber auch durch Polyaddition oder Polykondensation erhalten werden.

Die Einarbeitung der erfindungsgemäßen Farbkuppler in Silberhalogenidemulsionsschichten kann in der Weise erfolgen, daß zunächst von der betreffenden Verbindung eine Lösung oder eine Dispersion hergestellt und dann der Gießlösung für die betreffende Schicht zugefügt wird. Die Auswahl des geeigneten Lösungs- oder Dispersionsmittels hängt von der Löslichkeit der Verbindung ab.

Methoden zum Einbringen von in Wasser im wesentlichen unlöslichen Verbindungen durch Mahlverfahren sind beispielsweise in DE-A 26 09 741 und DE-A 26 09 742 beschrieben.

Hydrophobe Verbindungen können auch unter Verwendung von hochsiedenden Lösungsmittel, sogenannten Ölformern, in die Gießlösung eingebracht werden. Entsprechende Methoden sind beispielsweise in US-A 2 322 027, US-A 2 801 170 und EP-A 0 043 037 beschrieben. Anstelle von niedermolekularen Ölformern können auch Oligomere oder Polymere mit geeigneten Lösungsmitteleigenschaften Verwendung finden. Die erfindungsgemäßen Kuppler sind bevorzugt hydrophob ausgebildet.

Die Verbindungen können auch in Form sogenannter beladener Latices in die Gießlösung eingebracht werden. Verwiesen wird beispielsweise auf DE-A 25 41 230, DE-A25 41 274, DE-A 28 35 856, EP-A 0 014 921, EP-A 0 069 671, EP-A 0 130 115, US-A 4,291,113. Die diffusionsfeste Einlagerung anionischer wasserlöslicher Verbindungen (z.B. von Kupplern oder Farbstoffen) kann auch mit Hilfe von kationischen Polymeren, sogenannten polymeren Beizmitteln, erfolgen.

Geeignete Ölformer sind z.B. Phthalsäurealkylester, Phosphorsäureester, Phosphonsäureester, Zitronensäureester, Milchsäureester, Benzoesäureester, Fettsäureester, Amide, Alkohole, Phenole, Sulfonamide, Anilinderivate und Kohlenwasserstoffe.

Ein weiterer Gegenstand der vorliegenden Erfindung betrifft ein fotografisches Farbentwicklungsverfahren, bei dem man ein farbfotografisches Material, enthaltend eine Verbindung der Formel (I) in mindestens einer bildmäßig belichteten Silberhalogenidemulsion, mit einer Farbentwicklerverbindung vom Typ der Phenylendiamine und/oder Toluylendiamine entwickelt.

Typische im Sinn des erfindungsgemäßen Farbentwicklerverfahrens anwendbare und im ausgekuppelten Farbstoff wiederfindbare Farbentwicklerreste sind aus der Reihe der Phenylendiamine und/ oder Toluylendiamine wie beispielsweise 1-(N-Ethyl-N-methansulfonamido-ethyl)-3-methyl-p-phenylendiamin (CD 3), 1-(N-Ethyl-N-hydroxyethyl)-3-methyl-p-phenylendiamin (CD 4), 1-(N-Isopropyl-N-methansulfonamidoethyl)-3-methyl-p-phenylendiamin,1-(N-Isopropyl-N-hydroxyethyl)-3-methyl-p-phenylendiamin, 1-(N-Ethyl-N-3-hydroxypropyl)-3-methyl-p-phenylendiamin, N-Ethyl-N-4-sulfobutyl-3-methyl-p-phenylendiamin. Weitere brauchbare Farbentwickler sind beispielsweise in J.Am.Chem.Soc. 73, 3106 (1951) beschrieben.

Beim erfindungsgemäßen Verfahren wird ein farbfotografisches Material, das wenigstens einen bildmäßig belichtete Silberhalogenidemulsion enthält, bevorzugt mit einer Farbentwicklerverbindung vom Typ CD3 oder CD4 entwickelt. Die erfindungsgemäßen Farbkuppler sind dabei in räumlicher und spektraler Zuordnung zu einer farbsensibilisierten lichtempfindlichen Silberhalogenidemulsion im Material enthalten.

Unter räumlicher Zuordnung ist dabei zu verstehen, daß sich der Farbkuppler in einer solchen räumlichen Beziehung zu der betreffenden Silberhalogenidschicht befindet, daß eine Wechselwirkung zwischen ihnen möglich ist, die eine bildmäßige Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nicht lichtempfindlichen Bindemittelschicht.

Unter dem Begriff der spektralen Zuordnung ist zu verstehen, daß die Spektralempfindlichkeit der betreffenden lichtempfindlichen Silberhalogenidemulsion und die Farbe des aus dem räumlich zugeordneten Farbkuppler erzeugten Teilfarbenbildes in einer bestimmten Beziehung zueinander stehen, wobei normalerweise der Spektralempfindlichkeit jedes einzelnen Farbauszuges (Rot, Grün, Blau) ein komplementärfarbiges Teilfarbenbild (Blaugrün, Purpur, Gelb) zugeordnet ist. Entsprechend der aus den erfindungsgemäßen Blaugrün- und/oder Infrarotkupplern gebildeten Farbe werden diese vorzugsweise einer rotsensibilisierten Silberhalogenidemulsionsschicht zugeordnet.

Die erfindungsgemäß einzusetzenden Verbindungen der Formel (I) geben mit den Farbentwicklern des Standes der Technik Farbstoffe, deren Absorption entweder sehr nahe bei der Grenze zum Infrarotbereich oder schon darin, vorzugsweise im Bereich von 680 bis 750, insbesondere 700 bis 730 nm liegt. Kuppler, die zu Farbstoffen mit einer Absorption nahe 700 nm kuppeln, sind hervorragend als Blaugrünkuppler für CN-Filme geeignet. Solche, die zu Farbstoffen mit einer Absorption von mehr als 700 nm kuppeln, können als Infrarotkuppler bzw. sogenannte funktionale Kuppler eingesetzt werden, d.h. sie sind zur Abspaltung einer fotografisch wirksamen Gruppe befähigt, leisten aber keinen merklichen Beitrag zum farbigen Bildeindruck. Es ist jedoch ebenfalls möglich sie zur Erzeugung einer im Bild nicht visuell wahrnehmbaren Codierung, vorzugsweise im Papier, einzusetzen. Es könnten beispielsweise Daten zum Ursprung des Bildes oder Copyrightvermerke codiert werden. Die spektrale oder räumliche Zuordnung ist in diesem Fall an spezielle Funktionen des Kupplers gebunden. Im Fall von DIR-Kupplern erfolgt die spektrale oder räumliche Zuordnung zu einer Emulsionsschicht nach funktionellen Gesichtspunkten. Es ist z.B. sinnvoll, einen erfindungsgemäßen DIR-Kuppler in einer gesonderten Schicht unter dem rotempfindlichen Schichtpaket, bzw. Schichtanteil anzuordnen.

Ein weiterer Gegenstand der vorliegenden Erfindung betrifft die Verwendung von Verbindungen der Formel (I) als Blaugrün- bzw. Infrarotkuppler in farbfotografischen Silberhalogenidmaterialien.

Beispiele für farbfotografische Materialien sind Farbnegativfilme, Farbumkehrfilme, Farbpositivfilme, farbfotografisches Papier, farbumkehrfotografisches Papier, farbempfindliche Materialien für das Farbdiffusionstransfer-Verfahren oder das Silberfarbbleich-Verfahren. Eine Übersicht über typische farbfotografische Materialien sowie bevorzugte Ausführungsformen und Verarbeitungsprozesse findet sich in Research Disclosure 37038 (Februar 1995).

Die fotografischen Materialien bestehen aus einem Träger, auf den wenigstens eine lichtempfindliche Silberhalogenidemulsionsschicht aufgebracht ist. Als Träger eignen sich insbesondere dünne Filme und Folien. Eine Übersicht über Trägermaterialien und aufderen Vorder- und Rückseite aufgetragene Hilfsschichten ist in Research Disclosure 37254, Teil 1 (1995), S. 285 dargestellt.

Die farbfotografischen Materialien enthalten üblicherweise mindestens je eine rotempfindliche, grünempfindliche und blauempfindliche Silberhalogenidemulsionsschicht sowie gegebenenfalls Zwischenschichten und Schutzschichten.

Je nach Art des fotografischen Materials können diese Schichten unterschiedlich angeordnet sein. Dies sei für die wichtigsten Produkte dargestellt:

Farbfotografische Filme wie Colornegativfilme und Colorumkehrfilme weisen in der nachfolgend angegebenen Reihenfolge auf dem Träger 2 oder 3 rotempfindliche, blaugrünkuppelnde Silber-halogenidemulsionsschichten, 2 oder 3 grünempfindliche, purpurkuppelnde Silberhalogenid-emulsionsschichten und 2 oder 3 blauempfindliche, gelbkuppelnde Silberhalogenidemulsionsschichten auf. Die Schichten gleicher spektraler Empfindlichkeit unterscheiden sich in ihrer fotografischen Empfindlichkeit, wobei die weniger empfindlichen Teilschichten in der Regel näher zum Träger angeordnet sind als die höher empfindlichen Teilschichten.

Zwischen den grünempfindlichen und blauempfindlichen Schichten ist üblicherweise eine Gelbfilterschicht angebracht, die blaues Licht daran hindert, in die darunter liegenden Schichten zu gelangen.

Die Möglichkeiten der unterschiedlichen Schichtanordnungen und ihre Auswirkungen auf die fotografischen Eigenschaften werden in J. Inf. Rec. Mats., 1994, Vol. 22, Seiten 183 - 193 beschrieben.

Farbfotografisches Papier, das in der Regel wesentlich weniger lichtempfindlich ist als ein farbfotografischer Film, weist in der nachfolgend angegebenen Reihenfolge auf dem Träger üblicherweise je eine blauempfindliche, gelbkuppelnde Silberhalogenidemulsionsschicht, eine grünempfindliche, purpurkuppelnde Silberhalogenidemulsionsschicht und eine rotempfindliche, blaugrünkuppelnde Silberhalogenidemulsionsschicht auf; die Gelbfilterschicht kann entfallen.

Abweichungen von Zahl und Anordnung der lichtempfindlichen Schichten können zur Erzielung bestimmter Ergebnisse vorgenommen werden. Zum Beispiel können alte hochempfindlichen Schichten zu einem Schichtpaket und alle niedrigempfindlichen Schichten zu einem anderen Schichtpaket in einem fotografischen Film zusammengefaßt sein, um die Empfindlichkeit zu steigern (DE 2530645).

Wesentliche Bestandteile der fotografischen Emulsionsschichten sind Bindemittel, Silberhalogenidkörner und Farbkuppler.

Angaben über geeignete Bindemittel finden sich in Research Disclosure 37254, Teil 2 (1995), S. 286.

Angaben über geeignete Silberhalogenidemulsionen, ihre Herstellung, Reifung, Stabilisierung und spektrale Sensibilisierung einschließlich geeigneter Spektralsensibilisatoren finden sich in Research Disclosure 36544 (Sept.1994) und Research Disclosure 37254, Teil 3 (1995), S. 286 und in Research Disclosure 37038, Teil XV (1995), S. 89.

Fotografische Materialien mit Kameraempfindlichkeit enthalten üblicherweise Silberbromidiodidemulsionen, die gegebenenfalls auch geringe Anteile Silberchlorid enthalten können. Fotografische Kopiermaterialien enthalten entweder Silberchloridbromidemulsionen mit bis 80mol-% AgBr oder Silberchloridbromidemulsionen mit über 95mol-% AgCl.

Die fotografischen Emulsionen können unter Verwendung von Methinfarbstoffen oder anderen Farbstoffen spektral sensibilisiert werden. Besonders geeignete Farbstoffe sind Cyaninfarbstoffe, Merocyaninfarbstoffe, und komplexe Merocyaninfarbstoffe. Derartige Verbindungen, insbesondere Merocyanine, können auch als Stabilisatoren verwendet werden.

Eine Übersicht über die als Spektralsensibilisatoren geeigneten Polymethinfabstoffe, über deren geeignete Kombinationen und insbesondere über supersensibilisierend wirkende Kombinationen enthält Research Disclosure 17643 (1978), Kapitel IV, und Research Disclosure 18716 (1979), S. 648 (rechte Spalte) bis S. 649 (rechte Spalte).

Als Rotsensibilisatoren können darüberhinaus Pentamethincyanine mit Naphthothiazol, Naphthoxazol oder Benzthiazol als basische Endgruppen verwendet werden, welche mit Halogen, Methyl- oder Methoxygruppen substituiert und 9,11-alkylen-, insbesondere 9,11-neopentylen-verbrückt sein können wie in GB 604 217 und BE 660 948 beschrieben wird.Die N,N'-Substituenten können wie in EP 0 532 042 beschrieben auch C₄-C₈-Alkylgruppen sein. Die Methinkette kann zusätzlich noch Substituenten tragen wie in EP 0 532 042 erwähnt wird. Es können auch Pentamethine mit nur einer Methylgruppe am Cyclohexenring verwendet werden wie in EP 0 532 042 beschrieben wird. Der Rotsensibilisator kann, wie in BE 660 948 beschrieben, durch Zusatz heterocyclischer Mercaptoverbindungen supersensibilisiert und stabilisiert werden.

Die rotempfindliche Schicht kann zusätzlich zwischen 390 und 590 nm, bevorzugt bei 500 nm, wie in AG 5850/5851 beschrieben, spektral sensibilisiert sein, um so eine verbesserte Differenzierung der Rottöne zu bewirken, gemäß EP 0 304 297, US 806 460 und US 5 084 374.

Derartige Verbindungen, insbesondere Merocyanine, können auch als Stabilisatoren verwendet werden.

Die Spektralsensibilisatoren können in gelöster Form oder als Dispergat der fotografischen Emulsion zugesetzt werden. Sowohl Lösung als auch Dispergat können Zusätze wie beispielsweise Netzmittel oder Puffer enthalten.

Der Spektralsensibilisator oder eine Kombination von Spektralsensibilisatoren kann vor, während oder nach der Emulsionsbereitung zugesetzt werden.

Angaben zu den üblichen Farbkupplern finden sich in Research Disclosure 37254, Teil 4 (1995), S. 288 und in Research Disclosure 37038, Teil II (1995), S. 80. Die maximale Absorption der aus den Kupplern und dem Farbentwickleroxidationsprodukt gebildeten Farbstoffe liegt vorzugsweise in den folgenden Bereichen: Gelbkuppler 430 bis 460 nm, Purpurkuppler 540 bis 560 nm, Blaugrünkuppler 630 bis 700 nm.

In farbfotografischen Filmen werden zur Verbesserung von Empfindlichkeit, Körnigkeit, Schärfe und Farbtrennung häufig Verbindungen eingesetzt, die bei der Reaktion mit dem Entwickleroxidationsprodukt Verbindungen freisetzen, die fotografisch wirksam sind, z.B. DIR-Kuppler, die einen Entwicklungsinhibitor abspalten.

Angaben zu solchen Verbindungen, insbesondere Kupplern, finden sich in Research Disclosure 37254, Teil 5 (1995), S. 290 und in Research Disclosure 37038, Teil XIV (1995), S. 86.

Die meist hydrophoben Farbkuppler, aber auch andere hydrophobe Bestandteile der Schichten, werden üblicherweise in hochsiedenden organischen Lösungsmitteln gelöst oder dispergiert. Diese Lösungen oder Dispersionen werden dann in einer wäßrigen Bindemittellösung (üblicherweise Gelatinelösung) emulgiert und liegen nach dem Trocknen der Schichten als feine Tröpfchen (0,05 bis 0,8µm Durchmesser) in den Schichten vor.

Geeignete hochsiedende organische Lösungsmittel, Methoden zur Einbringung in die Schichten eines fotografischen Materials und weitere Methoden, chemische Verbindungen in fotografische Schichten einzubringen, finden sich in Research Disclosure 37254, Teil 6 (1995), S. 292.

Die in der Regel zwischen Schichten unterschiedlicher Spektralempfindlichkeit angeordneten nicht lichtempfindlichen Zwischenschichten können Mittel enthalten, die eine unerwünschte Diffusion von Entwickleroxidationsprodukten aus einer lichtempfindlichen in eine andere lichtempfindliche Schicht mit unterschiedlicher spektraler Sensibilisierung verhindern.

Geeignete Verbindungen (Weißkuppler, Scavenger oder EOP-Fänger) finden sich in Research Disclosure 37254, Teil 7 (1995), S. 292 und in Research Disclosure 37038, Teil III (1995), S. 84.

Das fotografische Material kann weiterhin UV-Licht absorbierende Verbindungen, Weißtöner, Abstandshalter, Filterfarbstoffe, Formalinfänger, Lichtschutzmittel, Antioxidantien, D_{Min}-Farbstoffe, Zusätze zur Verbesserung der Farbstoff-, Kuppler- und Weißenstabilität sowie zur Verringerung des Farbschleiers, Weichmacher (Latices), Biocide und anderes enthalten.

Geeignete Verbindungen finden sich in Research Disclosure 37254, Teil 8 (1995), S. 292 und in Research Disclosure 37038, Teile IV, V, VI, VII, X, XI und XIII (1995), S. 84 ff.

Die Schichten farbfotografischer Materialien werden üblicherweise gehärtet, d.h., das verwendete Bindemittel, vorzugsweise Gelatine, wird durch geeignete chemische Verfahren vernetzt.

Geeignete Härtersubstanzen finden sich in Research Disclosure 37254, Teil 9 (1995), S. 294 und in Research Disclosure 37038, Teil XII (1995), Seite 86.

Nach bildmäßiger Belichtung werden farbfotografische Materialien ihrem Charakter entsprechend nach unterschiedlichen Verfahren verarbeitet. Einzelheiten zu den Verfahrensweisen und dafür benötigte Chemikalien sind in Research Disclosure 37254, Teil 10 (1995), S. 294 sowie in Research Disclosure 37038, Teile XVI bis XXIII (1995), S. 95 ff. zusammen mit exemplarischen Materialien veröffentlicht.

### Beispiele

### Beispiel 1: Herstellbeispiel

### Vorstufe 1: Benzo[1,2,4]thiadiazin-3-essigsäure-(2-hexyl)decylester:

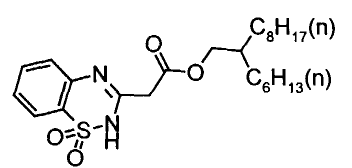

26,8 g Benzo[1,2,4]thiadiazin-1,1-dioxid-3-essigsäure-ethylester und 26,6 g 2-(n-Hexyl)decanol wurden in 10 ml o-Dichlorbenzol 4 Stunden unter Überleiten von Stickstoff auf 170°C erhitzt. Anschließend wurde das, Lösungsmittel im Hochvakuum abgedampft, in Toluol aufgenommen und über 300 g Kieselgel mit Toluol und steigenden Anteilen Dichlormethan als Laufmittel chromatografiert. Aus den polaren Eluaten erhielt man nach Eindampfen 39 g eines mit Farbentwickler/alkal. Persulfatlösung orangefarbig kuppelnden farblosen, bei 87 bis 90°C schmelzenden Kristallisats.

### Vorstufe 2: Benzo[1,2,4]thiadiazin-1,1-dioxid-3-isonitrosoessigsäure-(2-hexyl)-decylester:

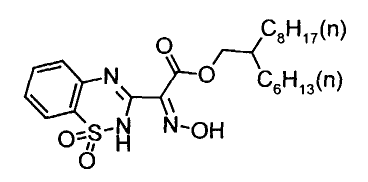

23,2 g Vorstufe 1 wurden in 160ml Essigsäure vorgelegt und unter Rühren bei 10 bis 20°C 7,0g Natriumnitrit so eingetragen, daß die Entwicklung von nitrosen Gasen vernachlässigbar blieb. Man rührte 30 Minuten bei Raumtemperatur nach, trug auf Eiswasser aus und rührte bis zur beendeten Kristallisation. Man saugte ab und trocknete das Produkt an der Luft. Man erhielt 20 bis 22 g eines bei 137 bis 140°C schmelzenden Festkörpers.

### Vorstufe 3: Benzo[1,2,4]thiadiazin-1,1-dioxid-3-formamidoessigsäure-(2-hexyl)-decylester:

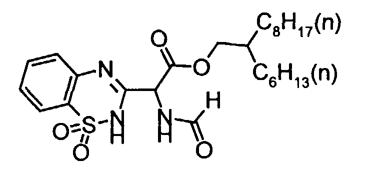

Man nahm 20g Vorstufe 2 in 150 ml Ameisensäure auf und erhitzte unter Zugabe von 20g Zinkstaub in kleinen Portionen zum Rückfluß. Nach 1 Stunde wurde vom Zinkstaub abdekantiert, auf 1000ml Eiswasser ausgetragen und dreimal mit je 200 ml Dichlormethan extrahiert. Die mit Wasser und Bicarbonatlösung neutralisierten Extrakte wurden eingeengt, der wachsartige Rückstand wurde ohne Reinigung weiterverarbeitet.

### Kuppler IRC-2:

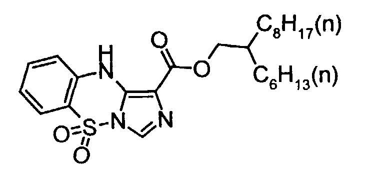

Man nahm den Eindampfrückstand von Vorstufe 3 mit 150 ml Toluol auf, gab 50 ml Phosphorylchlorid zu und erhitzte 1 Stunde auf Rückfluß. Man ließ auf Raumtemperatur abkühlen, goß die Lösung ohne Kühlung in 300 ml Wasser und wartete die stark exotherme Hydrolyse des überschüssigen Phosphorylchlorids ab. Man hielt noch 15 Minuten auf 65 bis 70°C, trennte die Toluolphase ab, wusch sie dreimal mit je 100 ml Wasser, trocknete mit Magnesiumsulfat und chromatografierte über 250g Kieselgel. Man erhielt aus den Eluaten der Hauptkomponente durch Eindampfen und Kristallisation aus Methanol 16,5g des Kupplers mit dem Schmelzpunkt 89 bis 91°C. Der Kuppler zeigte auf dem Dünnschichtchromatogramm mit Farbentwickler und alkalischer Persulfatlösung eine blaßblaugrüne Kupplung.

Das ¹H-NMR-Spektrum zeigt im Bereich der aromatischen und sauren Protonen:
ein Singulett (NH) bei 9,1 ppm,
ein Dublett bei 7,95 und 8,0 ppm
ein Singulett bei 7,84 ppm,
ein Triplett bei 7,62 - 7,74 ppm und ein nichtaufgelöstes Signal (A-B-System + einzelnes Proton) bei 7,18 - 7,35 ppm.

Die Entscheidung zugunsten der vorgelegen Struktur erfolgte über eine NOE-Differenz-Messung.

### Beispiel 2: Anwendungsbeispiel

Ein rotempfindlicher Teilschichtaufbau wurde hergestellt, indem auf einen transparenten Schichtträger aus Cellulosetriacetat folgende Schichten in der angegebenen Reihenfolge aufgetragen wurden. Die Mengenangaben gelten pro m². Die rotsensibilisierte Silberhalogenidemulsion ist mit 0,1 g bezogen auf 1Mol Silbernitrat 4-Hydroxy-6-methyl-1,3,3a,7-tetraazainden stabilisiert.

### Schichtaufbau 1.1.:

- Schicht 1:: (Antihaloschicht)
schwarzes kolloidales Silbersol aus
0,2 g AgNO₃
1,1 g Gelatine
- Schicht 2:: (Mikrat-Zwischenschicht)
AgBr-Mikratemulsion (mittlerer Korndurchmesser
0,07 µm) aus
0,25 g AgNO₃
1,0 g Gelatine
- Schicht 3: : rotempfindliche Schicht (kubische Silberbromidkristalle mit dem mittleren Korndurchmesser 0,5 µm) aus
5,35 g AgNO₃
3,5 g Gelatine
1,3 g Blaugrünkuppler C-1
1,3 g Trikresylphosphat
Schicht 4: (Härtung)
0,6 g Gelatine

0,73 g Soforthärtungsmittel SHM-1

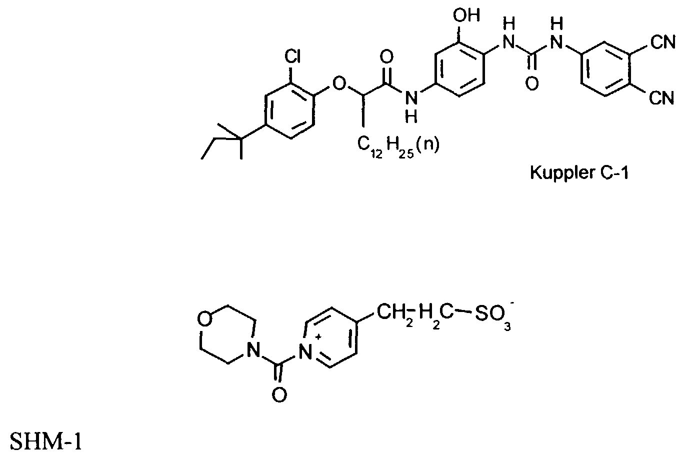

### Schichtaufbau 1.2.:

- Schicht 1:: (Antihaloschicht)
schwarzes kolloidales Silbersol
0,2 g AgNO₃
1,1 g Gelatine
- Schicht 2:: (Mikrat-Zwischenschicht)
AgBr-Mikratemulsion (mittlerer Korndurchmesser
0,07 µm) aus
0,25 g AgNO₃
1,0 g Gelatine
- Schicht 3:: (rotempfindliche Schicht) aus
4,5 g AgNO₃
3,0 g Gelatine
1,1 g erfindungsgemäßer Kuppler IRC-2
1,2 g Trikresylphosphat
- Schicht 4:: (Härtung)
0,55 g Gelatine
0,73 g Soforthärtungsmittel SHM-1

Die Proben wurden nach dem Beguß hinter einem Orangefilter und jeweils einem graduierten Graukeil mit Tageslicht belichtet und anschließend nach dem in "The British Journal of Photography" 1974, S.597 beschriebenen Prozeß verarbeitet.

Aus den Proben mit dem Schichtaufbau 1.1 (= Vergleich) erhielt man ein blaugrünes Bild, aus denen mit Schichtaufbau 1.2 (= erfindungsgemäß) ein ganz schwach bräunliches Bild mit einem Absorptionsmaximum bei 740 nm. Im Falle des erfindungsgemäßen Kupplers IRC-2 ist in der beschriebenen Einlagerungsform die Hauptabsorption so weit in das Infrarotgebiet verschoben, daß visuell praktisch kein Farbeindruck entsteht. Eine leichte Nebenabsorption bei 470 nm bedingt den schwach bräunlichen Farbton.

## Patentansprüche

1. Verbindung gemäß Formel (I) 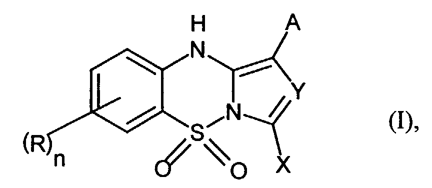 worin
A eine Elektronenakzeptorgruppe mit einem σₚ₋ Wert von> 0,25,
R einen Substituenten,
n 0,1,2,3 oder 4,
Y N und
X H oder eine bei der chromogenen Kupplung abspaltbare Gruppe
bedeuten.

2. Farbfotografisches Silberhalogenidmaterial mit wenigstens einer blauempfindlichen, wenigsten einen Gelbkuppler enthaltenden Silberhalogenidemulsionsschicht, wenigstens einer grünempfindlichen, wenigstens einen Purpurkuppler enthaltenden Silberhalogenidemulsionsschicht und wenigstens einer rotempfindlichen wenigstens einen Blaugrünkuppler enthaltenden Silberhalogenidemulsionsschicht, dadurch gekennzeichnet, daß der rotempfindlichen Schicht eine Verbindung der Formel (I) zugeordnet ist, 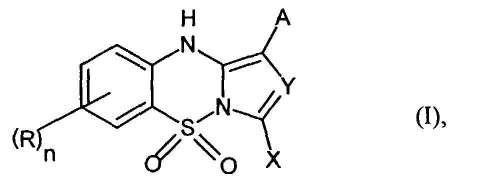 worin
A eine Elektronenakzeptorgruppe mit einem σₚ₋ Wert von> 0,25,
R einen Substituenten,
n 0,1,2,3 oder 4,
Y N oder C-CN und
X H oder eine bei der chromogenen Kupplung abspaltbare Gruppe
bedeuten.

3. Farbfotografisches Silberhalogenidmaterial nach Anspruch 2, dadurch gekennzeichnet, daß Y für N steht.

4. Farbfotografisches Silberhalogenidmaterial nach Anspruch 2 oder 3, dadurch gekennzeichnet, daß X für H, Halogen, einen Alkoxy-, Aryloxy-, Hetaryloxy-, Acyloxy-, Sulfonyloxy-, Alkoxycarbonyloxy-, Aryloxycarbonyloxy-, Alkylthio-, Arylthio-, Hetarylthio-, Alkoxycarbonylthio-, Acylalkylamino-, Alkoxycarbonylamino-, N-Alkylsulfonamido-, Aryloxycarbonylamino- oder Carboxysubstituenten sowie für einen über ein Stickstoffatom gebundenen heterocyclischen Rest oder eine Arylazo- oder Hetarylazogruppe oder den Rest einer fotografisch wirksamen Verbindung steht.

5. Farbfotografisches Silberhalogenidmaterial nach einem der Ansprüche 2 bis 4, dadurch gekennzeichnet, daß A für eine Alkoxycarbonyl- Carbamoyl- oder Cyangruppe steht.

6. Farbfotografisches Silberhalogenidmaterial nach einem der Ansprüche 2 bis 5, dadurch gekennzeichnet, daß (R)ₙ für -CN, -Cl, -OR¹, -NR²CO-O R¹, - CONH R¹, -COO R¹ oder -S R¹ steht, mit der Maßgabe, daß R¹ für eine Alkyl-, Aryl-, Arylen-, Hetaryl- oder Hetarylengruppe und R² für H oder einen Alkylrest steht.

7. Farbfotografisches Silberhalogenidmaterial nach einem der Ansprüche 2 bis 6, dadurch gekennzeichnet, daß n für 0,1 oder 2 steht.

8. Fotografisches Farbentwicklungsverfahren, dadurch gekennzeichnet, daß man ein farbfotografisches Material, enthaltend eine Verbindung der Formel (I) in mindestens einer bildmäßig belichteten Silberhalogenidemulsion, mit einer Farbentwicklerverbindung vom Typ der Phenylendiamine und/ oder Toluylendiamine entwickelt.

9. Fotografisches Farbentwicklungsverfahren nach Anspruch 8, dadurch gekennzeichnet, daß man ein farbfotografisches Material, enthaltend eine Verbindung der Formel (I) in mindestens einer bildmäßig belichteten Silberhalogenidemulsion, mit CD 3 entwickelt.

10. Verwendung von Verbindungen der Formel (I) als Blaugrün- und/oder Infrarotkuppler in farbfotografischen Silberhalogenidmaterialien.
